# EUROPEAN PATENT SPECIFICATION

(11) **EP 2 698 423 B1**
(45) Date of publication and mention of the grant of the patent: **23.12.2020**
(21) Application number: 12771036.6
(22) Date of filing: 13.04.2012
(51) Int. Cl.: C12M 1/00, C12M 1/06, B01D 15/08, B01D 15/22, C12P 7/16, C12P 7/06

(54) **APPARATUS AND METHOD FOR SEPARATING AND REFINING PRODUCT MANUFACTURED BY MICROBIAL FERMENTATION BY USING ADSORBENT**
VORRICHTUNG UND VERFAHREN ZUR TRENNUNG UND VERFEINERUNG EINES DURCH FERMENTIERUNG VON MIKROORGANISMEN HERGESTELLTEN PRODUKTS UNTER VERWENDUNG EINES ADSORPTIONSMITTELS
APPAREIL ET PROCÉDÉ DE SÉPARATION ET DE RAFFINAGE D`UN PRODUIT FABRIQUÉ PAR FERMENTATION DE MICRO-ORGANISMES À L`AIDE D`ADSORBANT

(30) Priority: 14.04.2011 KR 20110034883
(43) Date of publication of application: 19.02.2014
(73) Proprietor: GS Caltex Corporation, Seoul 135-985 (KR)
(72) Inventor: LEE, Sang-Hyun, Daejeon 305-762 (KR); EOM, Moon-HO, Seoul 121-856 (KR); LEE, Julia, Daejeon 305-728 (KR); JEON, Sang-Jun, Daejeon 305-728 (KR); CHO, Jung-Hee, Seongnam-si Gyeonggi-do 463-758 (KR); CHOI, Jin Dal Rae, Daejeon 305-701 (KR)
(74) Representative: Winter, Brandl, Fürniss, Hübner, Röss, Kaiser, Polte - Partnerschaft mbB
(86) International application number: PCT/KR2012/002845
(87) International publication number: WO 2012/141546

(56) References cited:
- EP-A2- 0 151 470
- EP-B1- 1 087 828
- WO-A1-86/00339
- WO-A1-2008/095896
- WO-A1-2009/036076
- CN-A- 101 914 433
- CN-B- 101 130 791
- JP-A- 2006 333 749
- KR-A- 20010 046 516

## Description

### [Technical Field]

The present invention relates to an apparatus and a method for continuously separating and refining products produced by microbial fermentation.

### [Background Art]

Butanol can be used as a chemical intermediate in cosmetics, perfume, hormone, a sanitizer, an industrial coating agent, a paint additive, fiber, a plastic monomer, medical supplies, vitamins, antibiotics, pesticides, or the like (Document [Durre, Biotechnol. J, 2:1525-1534, 2007]).

As the existing method for preparing butanol, a method of fermenting sugar using *Clostridium* strain to produce butanol, acetone, and ethanol (Document [US Patent Laid-Open Publication No. 1,315,585]) has been used until the 1980s, but thereafter, an oxo process of synthesizing butanol from propylene obtained from petroleum has been widely used. However, since the method for preparing butanol based on petroleum is complicated due to using high temperature and high pressure and large amounts of hazardous wastes and carbon dioxide are discharged (Document [Tsuchida et al., Ind. Eng. Chem. Res., 45: 8634, 2006]). Recently, a demand for eco-friendly producing butanol from sustainable resources through microbial fermentation has been increased again.

However, as described above, currently, in most cases, butanol has been produced by a chemical synthesis method. An interest in research into biobutanol has been rapidly increased around the world due to an increase in oil price, environmental problems, and the like, but biobutanol has not yet been efficiently produced.

In the case of butanol, up to now, in most of the examples of producing butanol through fermentation, *Clostridium* strain is used. There is an example in which productivities of acetone, butanol, and ethanol were increased by 95%, 37%, and 90%, respectively, as compared to the case of wild-type strains by inserting three genes, that is, acetoacetic acid decarboxylase (adc), CoA transferase A (ctfA), and CoA transferase B (cftB), into a vector and using a promoter of adc to thereby construct an artificial operon and then introducing this plasmid pFNK6 into *Clostridium acetobutylicum* ATCC 824 strains (Document [Mermelstein et al., Biotechnol. Bioeng., 42:1053, 1993]). In addition, there is an example in the recombinant strain cloning and expressing alcohol/aldehyde dehydrogenase(aad) produced higher amount of butanol and ethanol than aceton when compared with wild type (Document [Nair et al., J. Bacteriol., 176:871, 1994]). Otherwise, as a method for inactivating a function of genes, there is an example of inactivating butyrate kinase (buk) and phosphotransacetylase (pta). It was reported that when a strain PJC4BK in which the buk gene was inactivated was fermented at pH of 5.0 or more, a production amount of butanol was significantly increased up to 16.7 g/L (Document [Harris et al., Biotechnol. Bioeng., 67:1, 2000]). However, it was reported that when the case of a strain in which a pta gene was inactivated was compared with the case of a wild-type strain, there was no significant difference in producing a solvent (Document [Harris et al., Biotechnol. Bioeng., 67:1, 2000]). In addition, it was reported that when fermentation was performed using a *Clostridium beijerinckii* BA101 strain, which is a mutant strain induced by random mutation, and maltodextrin as a carbon source, 18.6 g/L of butanol was produced (Document [Ezeji et al., Appl. Microbiol. Biotechnol., 63:653, 2004]). However, even in the case of using these recombinant strains, the production amount of butanol in a culture medium was significantly low (20 g/L or less) due to toxicity of butanol, which is a final product, such that it was impossible to industrially use these recombinant strains. Therefore, various method for extracting butanol *in situ* produced during a culture process to maintain a concentration of butanol in a culture medium at a level at which cytotoxicity is not generated have been developed. For example, it was reported that productivity may be increased by adsorbing butanol produced during continuous culture using activated carbon (Document [US Patent Registration No. 4520104]).

However, in this method, only butanol is selectively adsorbed by the activated carbon and a concentration of the adsorbed butanol is low, such that it is difficult to recover butanol, and physical stability of activated carbon is insufficient, such that it is impossible to reuse the activated carbon. Therefore, there is a disadvantage in that extraction of butanol is expensive. An adsorption amount of butanol is in proportion to a concentration of butanol, but the concentration of butanol produced in continuous culture is low, such that the adsorption amount of butanol is also significantly low. Due to this problem, in spite of the continuous culture process, the productivity is not over 1 g/L/h. In addition, a column filled with the activated carbon may be physically clogged due to aggregation of cells, thereby causing a problem in a process. In this method, cell aggregates may clog the column and form a channel in a flow of the culture medium, such that it is difficult to allow the products such as butanol, acetone, isopropanol, ethanol, or the like, to be adsorbed in the entire adsorbent, thereby decreasing adsorption efficiency. A method of using an adsorbent except for activated carbon to increase productivity and a concentration of a solvent and using a recyclable adsorbent has been reported (Document [Nielsen et al., Bioeng. Biotech. 102:811-821, 2009]). The method is a method of adding the adsorbent in a culture medium and adsorbing butanol produced during a culture process in the adsorbent to recover butanol. According to this method, the adsorbent should be recovered from the culture medium, and essentially, a loss of the adsorbent is generated in a recovering process. In addition, impurities produced by microbes in the culture process and sugar, which is a raw material, are simultaneously adsorbed, such that purity and productivity of butanol are low at the time of recovering butanol. Further, an adsorption amount of butanol is in proportion to an amount of adsorbent added to the culture medium, but in this method, there is a limitation in an addition amount of the adsorbent in the culture medium. In addition, in this method, concentrations of ethanol and acetone are relatively high, which serves to desorb the adsorbed butanol, such that there is a limitation in increasing the concentration of butanol.
CN101914433 discloses a lactic acid production device for fermentation and separation coupling and a lactic acid fermentation process using expanded bed in-situ adsorption to realize online separation. An expanded bed and a fermentation tank are coupled, the online separation of lactic acid fermentation is realized by using the expanded bed method, so that the feedback inhibition of lactic acid is relieved, the yield and the conversion rate of the lactic acid fermentation are increased.

### [Disclosure]

### [Technical Problem]

An object of the present invention is to provide a separation and refinement apparatus capable of continuously separating and refining products produced from a fermented culture medium of a microbe, and a separation and refinement method.

### [Technical Solution]

In one general aspect, there is provided a continuous fermentation, separation, and refinement apparatus of products including:
a culture bath in which a microbe is cultured together with a raw material to produce the products;
at least two columns filled with an adsorbent; and
a conversion part configured to control a flow of a culture medium containing the products, so that a culture medium containing the products is supplied from the culture bath to a specific column,
wherein the conversion part is configured to change into where the culture medium flows from a first to a second column in the case in which the products are sufficiently adsorbed in the first column supplied with the culture medium, wherein a continuous flow of culture medium from the culture bath is maintained, wherein
the case in which the products are sufficiently adsorbed is a case in which an adsorption rate of the product with respect to the adsorbent is decreased, a case in which a concentration of the product in the discharge solution discharged from the column is 80% or more of a concentration of the product in the culture medium supplied to the column, a case in which growth of the microbe in the culture bath is inhibited by an increase in the concentration of the product, or a case in which productivity of the product of the microbe is decreased, or a case in which the concentration of butanol in the culture medium reaches at about 112 g/L,
and wherein the first column and second column each include a stirrer stirring the adsorbent and the product in the column and a filter preventing the adsorbent from being eluted to thereby be lost.

In another aspect of the present invention, there is provided a continuous fermentation, separation, and refinement method of products including:
culturing a microbe together with a raw material to produce the products;
supplying a culture medium containing the products to a first column filled with an adsorbent; and
stopping supplying the culture medium to the first column in the case in which the products are sufficiently adsorbed in the first column and supplying the culture medium to a second column wherein a continuous flow of culture medium from the culture bath is maintained,
wherein at least some of the discharge solution discharged from the first column is used in culturing the microbe again, and wherein
the case in which the products are sufficiently adsorbed is a case in which an adsorption rate of the product with respect to the adsorbent is decreased, a case in which a concentration of the product in the discharge solution discharged from the column is 80% or more of a concentration of the product in the culture medium supplied to the column, a case in which growth of the microbe in the culture bath is inhibited by an increase in the concentration of the product, or a case in which productivity of the product of the microbe is decreased, or a case in which the concentration of butanol in the culture medium reaches at about 112 g/L.

### [Advantageous Effects]

With the fermentation, separation, and refinement apparatus and method according to the present invention, products in the culture medium of a microbe may be simply and rapidly separated and purified.

### [Description of Drawings]

FIG. 1 is a conceptual diagram showing a fermentation, separation, and refinement apparatus according to the present invention.
FIG. 2 is a comparison graph showing relative butanol adsorption performance of adsorbents.
FIG. 3 shows kinetic analysis of butanol adsorption rate to the adsorbent with various concentrations.
FIG. 4 shows fermentation profile obtained by performing culture using a culture method according to the exemplary embodiment of the present invention (ABE: Acetone, butanol, and ethanol).

### [Best Mode]

Advantages and features of the present invention and methods to achieve them will be elucidated from exemplary embodiments described below in detail with reference to the accompanying drawings. However, the present invention is not limited to the preferred embodiment disclosed herein but will be implemented in various forms. The preferred embodiments make disclosure of the present invention thorough and are provided so that those skilled in the art can easily understand the scope of the present invention. Therefore, the present invention will be defined by the scope of the appended claims. Like reference numerals throughout the description denote like elements.

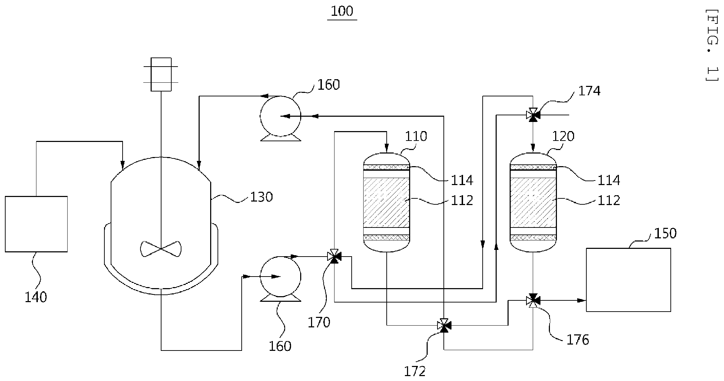

The present invention relates to a continuous fermentation, separation, and refinement apparatus of products including:
a culture bath in which a microbe is cultured together with a raw material to produce the products;
at least two columns filled with an adsorbent; and
a conversion part configured to control a flow of a culture medium containing the products, so that a culture medium containing the products is supplied from the culture bath to a specific column,
wherein the conversion part is configured to change into where the culture medium flows from a first to a second column in the case in which the products are sufficiently adsorbed in the first column supplied with the culture medium, wherein a continuous flow of culture medium from the culture bath is maintained, wherein
the case in which the products are sufficiently adsorbed is a case in which an adsorption rate of the product with respect to the adsorbent is decreased, a case in which a concentration of the product in the discharge solution discharged from the column is 80% or more of a concentration
of the product in the culture medium supplied to the column, a case in which growth of the microbe in the culture bath is inhibited by an increase in the concentration of the product, or a case in which productivity of the product of the microbe is decreased, or a case in which the concentration of butanol in the culture medium reaches at about 112 g/L,
and wherein the first column and second column each include a stirrer stirring the adsorbent and the product in the column and a filter preventing the adsorbent from being eluted to thereby be lost(FIG. 1).

In addition, the present invention relates to a continuous fermentation, separation, and refinement method of products including:
culturing a microbe together with a raw material to produce the products;
supplying a culture medium containing the products to a first column filled with an adsorbent; and
stopping supplying the culture medium to the first column in the case in which the products are sufficiently adsorbed in the first column and supplying the culture medium to a second column wherein a continuous flow of culture medium from the culture bath is maintained,
wherein at least some of the discharge solution discharged from the first column is used in culturing the microbe again, and wherein
the case in which the products are sufficiently adsorbed is a case in which an adsorption rate of the product with respect to the adsorbent is decreased, a case in which a concentration of the product in the discharge solution discharged from the column is 80% or more of a concentration of the product in the culture medium supplied to the column, a case in which growth of the microbe in the culture bath is inhibited by an increase in the concentration of the product, or a case in which productivity of the product of the microbe is decreased, or a case in which the concentration of butanol in the culture medium reaches at about 112 g/L.

Further, the present invention relates to a continuous fermentation, separation, and refinement method of products including:
culturing a microbe together with a raw material to produce the products;
supplying a culture medium containing the products to a first column filled with an adsorbent;
stopping supplying the culture medium to the first column in the case in which the products are sufficiently adsorbed in the first column and supplying the culture medium to a second column; and
desorbing the adsorbed products from the adsorbent in the first column to which the culture medium is not supplied while the products are adsorbed in the second column to which the culture medium is supplied.

Hereinafter, the fermentation, separation, and refinement apparatus of products produced by microbial fermentation according to the present invention (hereinafter, referred to as the 'fermentation, separation, and refinement apparatus') and the fermentation, separation, and refinement method of products produced through microbial fermentation according to the present invention (hereinafter, referred to as the 'fermentation, separation, and refinement method') will be described in detail with reference to the accompanying drawings.

The fermentation, separation, and refinement apparatus according to the present invention includes at least two columns filled with the adsorbent. The fermentation, separation, and refinement apparatus according to the present invention includes, preferably, 2 to 20 columns filled with the adsorbent, more preferably, 2 to 10 columns, further more preferably, 2 to 5 columns, and most preferably, 2 to 3 columns. These columns may be represented by a first column, a second column, a third column, a fourth column, a fifth column, a sixth column, or the like. However, for convenience of explanation, hereinafter, the present invention will be described based on two columns. As used herein, the term "the first and second columns" means arbitrarily numbered columns for convenience of explanation, but does not mean that the fermentation, separation, and refinement apparatus includes only two columns. In addition, according to the present invention, the culture medium is continuously supplied to several columns, such that a description of one column is similarly applied to other columns.

In a culture bath 130, the microbe is cultured by being supplied with the raw material and a medium from a supply bath 140. As a result, in the culture bath 130, the products are produced in the fermented culture medium(hereinafter, referred to as a 'culture medium') by microbial fermentation.

The culture medium discharged from the culture bath 130 is supplied to a first column 110 using a pump 160. The culture medium may be continuously or discontinuously supplied. The product produced by microbe fermentation is contained in the culture medium supplied to the first column 110, and the product in the culture medium is adsorbed in an adsorbent 112 in the column.

In this case, the fermentation, separation, and refinement apparatus according to the present invention may further include a stirrer (not shown) stirring the adsorbent and the product in the column. The culture medium and the adsorbent in the first column 110 are uniformly mixed with each other using the stirrer, thereby making it possible to prevent the culture medium and the adsorbent from being aggregated in the column, particularly, at a portion to which the culture medium is supplied, a portion at which a discharge solution is discharged outside of the column, or the like. The culture medium may circulate from an upper portion of the first column 110 to a lower portion thereof, but is not limited thereto.

When the product is sufficiently adsorbed in the first column 110, connection between the culture bath 130 and the first column 110 is blocked by a first conversion part 170, such that supply of the culture medium to the first column 110 is stopped. Further, the first conversion part 170 and a third conversion part 174 are opened in a direction in which the culture bath 130 and a second column 120 are connected to each other, such that the culture medium is supplied to the second column 120. That is, when the product is sufficiently adsorbed in the first column 110, a flow of the culture medium is changed so that supply of the culture medium to the first column 110 is stopped and the culture medium is supplied to the second column 120. At this time, the first and third conversion parts 170 and 174 may include a 4-way valve.

While adsorption is performed in the second column 120 to which the culture medium is supplied, desorption is performed in the first column 110 to which supply of the culture medium is stopped. The term "desorption" means to elute the product adsorbed in the adsorbent from the adsorbent to allow the adsorbent to be recyclable.

The desorption may be performed using heat or an eluant, but is not limited thereto. In addition, a kind of means used in the desorption is not limited as long as it is suitable for a process condition. The desorption may be performed anytime by using heat or the eluant, and a sufficiently large amount of product may be separated and purified with a small amount of adsorbent 112.

For example, desorption in the present invention may be performed by applying heat to the adsorbed product to vaporize the product. In this case, in view of cost, the heat generated in a process is preferable, and the heat may be applied as steam or hot air.. The steam may be water in a vapor state and applied at a pressure of 0.01 to 6 bar. In addition, the hot air may be applied at a pressure of 0.01 to 6 bar and have a temperature at which the product may be eluted in a state in which the column and the adsorbent are not damaged. For example, the temperature of the hot air may be 100 to 200°C, preferable 110 to 150°C, more preferably 120 to 140°C, and most preferably 130°C. In addition, the desorption in the present invention may be performed using the eluant, and the eluant may be an organic solvent, or an acidic or basic aqueous solution. The organic solvent may be tetrahydrofuran, alcohol, ketone, ether, or ester, preferably, methanol, acetone, ethylacetate, diethylether, or methylethylketone, but is not limited thereto.

As an example of a desorption method, a tetrahydrofuran solvent is flowed into the column or water vapor is passed through the column. For example, desorption may be performed by flowing the tetrahydrofuran solvent having a double volume of a volume of the adsorbent at a flow rate of 10mL/min or passing vapor preferably at 130°C and 2 bar.

The desorption proceeds in a state in which the adsorbent in the first column 110 is not picked out, that is, an in-situ state. As described above, when the product is adsorbed in-situ in the adsorbent 112, butanol in the culture medium present in the first column 110 is maintained at a concentration at which butanol does not inhibit growth of the microbe or productivity of the product. While the desorption proceeds in the first column 110, the culture medium is supplied to the second column 120, such that the culture medium may be continuously flowed. In addition, in the second column 120 to which the culture medium is supplied, adsorption continuously proceeds, such that fermentation, separation, and refinement of the product may be continuously performed.

The fermentation, separation, and refinement apparatus according to the present invention may further include a filter 114 at the upper or lower portion of the column in order to prevent the adsorbent 112 from being eluted to thereby be lost.

The case in which the product in the column is sufficiently adsorbed means a case in which the product is sufficiently adsorbed in the adsorbent in the column. That is, the product is sufficiently adsorbed, which means that since the product is sufficiently adsorbed in the first column, it is judged that the case of stopping adsorption of the product in the first column and adsorbing the product in the second column is more preferable as compared to the case of producing and adsorbing the product in the first column.

For example, the case in which the product of the present invention is sufficiently adsorbed may be the case in which an adsorption rate of the product with respect to the adsorbent is decreased or the case in which a concentration of the product in a discharge solution discharged from the column is 80% or more of a concentration of the product in the culture medium supplied to the column. Meanwhile, the product produced by the microbe, particularly, butanol is contained in the fermented culture medium of the microbe of the present invention. When the concentration of butanol in the culture medium reaches at about 12g/L, the microbe may be fatally affected by toxicity of butanol. However, at least some of the discharge solution discharged from the column of the present invention is supplied to the culture bath, and as the discharge solution is continuously supplied to the culture bath, the product in the culture bath, particularly butanol is accumulated, such that the concentration of butanol is increased, thereby inhibiting culture of the microbe in the culture bath. Therefore, the case in which the product of the present invention is sufficiently adsorbed may be the case in which the growth of the microbe in the culture bath is inhibited by an increase in the concentration of the product or the case in which productivity of the product of the microbe is decreased.

As a result, the case in which the product is sufficiently adsorbed may be the case in which the adsorption rate of the product with respect to the adsorbent is decreased, the case in which the concentration of the product in the discharge solution discharged from the column is 80% or more of the concentration of the product in the culture medium supplied to the column, the case in which the growth of the microbe in the culture bath is inhibited by the increase in the concentration of the product, or the case in which productivity of the product of the microbe is decreased, and this case may be suitably determined by those skilled in the art according to the kind of microbe, the kind of adsorbent, the kind and composition of product, and the like.

Thereafter, when the product is sufficiently adsorbed in the second column 120, connection between the culture bath 130 and the second column 120 is blocked by the first and third conversion parts 170 and 174, such that supply of the culture medium to the second column 120 is stopped. Then, the conversion part is opened in a direction in which the culture bath 130 and a different column are connected to each other, such that the culture medium is supplied to the different column. In addition, desorption proceeds in the second column 120, and the product in the culture medium is adsorbed in the different column, such that separation and refinement of the product is continuously performed. In this case, desorption in the second column 120 proceeds similarly to desorption in the first column 110.

The "different column' may be the first column 110 or a third column. The different column may be changed according to the number of columns included in the fermentation, separation, and refinement apparatus. That is, in the case in which the number of columns in the fermentation, separation, and refinement apparatus is 3 or more, the "different column' is a third column. In the case in which the number of columns in the fermentation, separation, and refinement apparatus is 2, the "different column' is the first column 110, and the first column 110 is in a state in which desorption is completed while the adsorption of the product in the second column 120 proceeds. In this case, the first conversion part 170 is opened in the direction in which the culture bath 130 and the first column 110 are connected to each other, such that the culture medium is supplied to the first column 110. In addition, while desorption proceeds in the second column 120, adsorption of the product proceeds in the first column 110. The fermentation, separation, and refinement apparatus according to the present invention may reuse the adsorbent in the columns by repeating the above-mentioned process and continuously ferment, separate, and purify the product.

Meanwhile, the fermentation, separation, and refinement apparatus according to the present invention may further include a storage bath 150 storing the product desorbed from the adsorbent filled in the first column 110. While desorption proceeds in the first column 110, second and fourth conversion parts 172 and 176 are opened in a direction in which the storage bath 150 and the first column 110 are connected to each other. Therefore, the product desorbed from the first column 110 moves to the storage bath 150. In this case, the second and fourth conversion parts 172 and 176 may include a 4-way valve.

In the case in which desorption proceeds in the second column 120, the desorbed product may also be stored in the storage bath 150. In this case, the fourth conversion part 176 is opened in a direction in which the storage bath 150 and the second column 120 are connected to each other, thereby making it possible to move the desorbed product to the storage bath 150.

Meanwhile, at least some of the discharge solution discharged from the column of the present invention may be supplied to the culture bath. In this case, an amount of discharge solution supplied to the culture bath may be equal to or less than an amount of culture medium supplied to the column.

Hereinabove, the fermentation, separation, and refinement apparatus and method of the present invention are briefly described. Hereinafter, the present invention will be described in detail.

The fermentation, separation, and refinement apparatus and method according to the present invention may simply and continuously ferment, separate, and purify the product produced by culturing the microbe, and since desorption may be suitably performed when the product is sufficiently adsorbed in the adsorbent, adsorption efficiency may also be high. Therefore, the fermentation, separation, and refinement apparatus and method according to the present invention may separate and purify the product produced by culturing the microbe with the high efficiency.

As the microbe of the present invention, any microbe may be used as long as the microbe may produce the product, which is a biofuel, from the raw material, but the present invention is not particularly limited. For example, the microbe of the present invention may be bacteria, yeast, fungus, or the like, and preferably, bacteria or yeast. The microbe of the present invention may be a wild-type microbe or genetically modified microbe. Preferably, the microbe of the present invention may be a wild type strain of *Clostridium* genus, *E. coli,* or the like, or a strain of which genes are recombined so as to increase productivity of a specific product, but it is obvious to those skilled in the art that the present invention is not limited thereto.

For example, in the case of using bacteria in the *Clostridium* genus of which buk gene coding butyrate kinase is deleted as the microbe of the present invention, there is an advantage in that products such as butanol, acetone, isopropanol, ethanol, or the like, may be produced at a high concentration under anaerobic conditions while not producing a large amount of butyric acid. For example, in the case of using bacteria in the *Clostridium* genus of which buk gene coding butyrate kinase, pta gene coding phosphotransacetylase, and ackA gene coding acetate kinase are deleted as the microbe of the present invention, there is an advantage in that products such as butanol, acetone, isopropanol, ethanol, or the like, may be produced at a high concentration under anaerobic conditions while not producing large amounts of other organic acids. In addition, in the case of using bacteria in the *Clostridium* genus of which adc gene coding acetoacetic acid decarboxylase is deleted as the microbe of the present invention, there is an advantage in that butanol may be selectively produced at a high concentration while not producing acetone. Further, in the case of using bacteria in the *Clostridium* genus transformed with a plasmid including genes coding secondary alcohol dehydrogenase (ald), which is an enzyme converting acetone into isopropylalcohol, there is an advantage in that high value-added products such as butanol, isopropanol, ethanol, or the like, may be produced.

The gene modified *Clostridium* may be prepared by a method disclosed in the released paper (Document [Microbiology (1996), 142, 2079-2086]). For example, PJC4BK or BKM19(KCTC 10558BP), which is a mutant strain, may be prepared by deleting the butyrate kinase gene (buk) from *Clostridium acetobutylicum.* Thereafter, mutant strains may be prepared by deleting the phosphotransacetylase gene (pta) and the acetate kinase gene (ackA) from the strain, respectively. Next, it may be confirmed that when these strains are cultured under anaerobic conditions, the products such as butanol, acetone, isopropanol, ethanol, or the like, are produced at a high concentration.

The microbe of the present invention may be cultured by a fed-batch culture method or continuous culture method. The continuous culture method is a method of supplying a fresh medium to a culture bath at a predetermined rate and simultaneously discharging the same amount of fermented culture medium of the microbe to thereby always maintain the culture medium to be constant in the culture bath. Meanwhile, the fed-batch culture method, which is a culture method of intermittently supplying a medium, is a method of optionally controlling a concentration of a substrate in the culture medium.

The raw material of the present invention may be a material capable of being used by the microbe at the time of fermentation to thereby produce the product, and a kind thereof is not particularly limited. For example, the raw material of the present invention may be biomass, sugars, fatty acids, or the like. The biomass may be a woody biomass, grains, or the like, but is not limited thereto. The sugars may be monosaccharides, polysaccharides, polysaccharides, or the like, be (C3-C12) sugar, and include polysaccharides produced during a hydrolysis process of the biomass. For example, the sugars may be glycerol, glucose, sucrose, xylose, starch, cellulose, or the like, but is not limited thereto. The fatty acid may be (C2-C24) fatty acid, for example, acetic acid, butyric acid, propionic acid, or the like, but is not limited thereto.

The product of the present invention is a fermented product produced by the microbe. Preferably, the product of the present invention may be alcohol, ketone, ester, carboxylic acid, or the like, as a biofuel, but is not limited thereto. For example, the alcohol may be (C2-C6)alcohol, preferably, alcohol having at most 4 carbon atoms, and more preferably, ethanol, propanol, isopropanol (2-propanol), 1,2-propanediol, 1,3-propanediol, 1,3-butanediol, 1,4-butanediol, butanol, or the like, but is not limited thereto. The ketone may be (C3-C8)ketone. For example, the ketone may be acetoacetate, acetone, or the like, but is not limited thereto. The ester may be (C4-C8)ester, for example, ethyl acetate, ethyl butyrate, butyl acetate, butyl butyrate, or the like, but is not limited thereto. The carboxylic acid may be (C2-C8)carboxylic acid, for example, acetic acid, butyric acid, propionic acid, or the like, but is not limited thereto. Preferably, the product of the present invention may be butanol, isopropanol, ethanol, or acetone, but is not limited thereto.

The adsorbent 112 of the present invention may be filled at a volume fraction of 0.1 to 99% based on a capacity, that is, a volume of the column containing the adsorbent 112. In the case in which the volume fraction of the adsorbent is less than 0.1 volume% based on the volume of the column, the amount of the adsorbent 112 is insufficient, such that adsorption may not be suitably performed. Further, in the case in which the volume fraction of the adsorbent is 99 volume% or more, aggregates of the adsorbent 112 or cells are formed in the column, such that adsorption may not be suitably performed.

A type of column to which the culture medium is supplied may be a slurry reactor type, a fluidized reactor type, or a packed reactor type. The fluidized reactor type column is a column containing a small amount of an adsorbent and having high fluidity, the slurry reactor type column is a column containing an adsorbent suspended in a culture medium, and the packed reactor type column is a column in which an adsorbent is substantially packed. In this case, the middle type columns of the three type columns may be divided into the three type columns depending on which column they are close to, and although the inside of the column is the middle type of the three types, the column may be included in any one of the three types. For example, according to the degree of fluidity, when it is judged that the adsorbent is packed so as not to substantially flow, the column may be classified as the packed type column, and when it is judged that the adsorbent may be suspended, the column may be classified as the slurry reactor type column.

The conversion part of the present invention serves to change and control the flow of the culture medium or discharge solution and may control so that the culture medium is supplied from the culture bath 130 to a specific column or send the discharge solution discharged from a specific column to the storage bath 150. A single or a plurality of conversion parts may be used, and the conversion part may be suitably installed by those skilled in the art according to the design of the fermentation, separation, and refinement apparatus of the present invention.

### <Experimental Example 1>

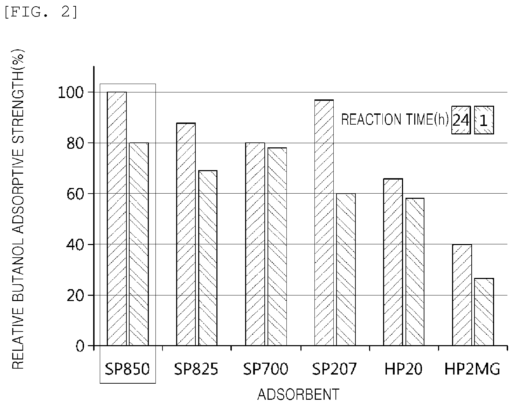

In order to select a suitable adsorbent used in the Example of the present invention, butanol adsorption performance of various adsorbents prepared by Mitsubishi Corp. was compared. After various kinds of adsorbents were added to 50mL of a phosphate buffer solution (50mM) containing 2% butanol and left for 1 hour while being stirred, a concentration of butanol remaining in the solution was analyzed using gas chromatography. As a result, it was analyzed that the adsorbent SP850 had most excellent performance (FIG. 2), but the kind of adsorbent is not limited to SP850. Analysis of products such as butanol, acetone, isopropanol, ethanol, or the like, was performed using the gas chromatography (Agilent, USA), and analysis conditions were as shown in the following Table 1.

### Table 1

**[Table 1]**

| | |
|---|---|
| Injector temperature | 320°C |
| detector temperature | 320°C |
| Injector split ratio | 20/1 |
| Injection volume | 0.1µL |
| Oven conditions | 80°C/20min |
| Air flux | 300mL/min |
| H2 flux | 30mL/min |
| Column: Supelco CarboWAX | |

Gas chromatography analysis condition

### <Experimental Example 2>

In order to confirm an adsorption rate of butanol adsorbed in the adsorbent SP850, kinetic analysis was performed on adsorption of butanol. In the culturing used in Example of the present invention, since butanol should be adsorbed for a short time for which the culture medium passed through the column, the adsorption rate of butanol was significantly important. First, 3g (dried weight) of the adsorbent was added to 50mL of liquid *Clostridium* growth media (CGM) in which butanol was contained at various concentrations, and sampling was performed at sample times of 30 seconds, 1 minute, 2 minutes, 5 minutes, 10 minutes, 30 minutes, and 1 hour while stirring the mixture at 200 rpm. Then, a concentration of butanol remaining in the solution was confirmed through gas chromatography analysis.

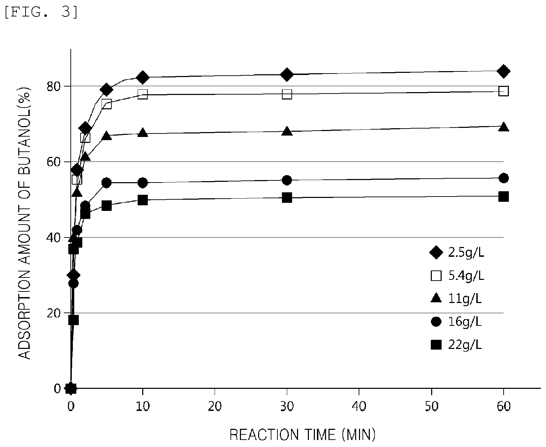

As a result, it may be confirmed that butanol was adsorbed within about 1 minute in various concentration ranges (FIG. 3).

### <Experimental Example 3>

In order to confirm an amount of adsorbent suitable of fed-batch fermentation and fermentation conditions, a recombinant *Clostridium* strain was constructed. A recombinant *Clostidium* strain (*C*. *acetobutylicum* PJC4BK-IPA2) containing secondary alcohol dehydrogenase required for fermenting the microbe was constructed. The *Clostridium* strain was cultured in 60mL of liquid *Clostridium* growth media (CGM, 0.75g/L K2HPO4, 0.75g/L KH2PO4, 0.7g/L, MgSO4H2O, 0.017g/L MnSO4H2O, 0.01g/L, FeSO4H2O, 2g/L (NH4)2SO4, 1g/L NaCl, 2g/L asparagine, 0.004g/L p-aminobenzoic acid, 5g/L yeast extract, 4.08g/L CH3COONaH2O, and 80g/L glucose) under anaerobic conditions until OD600 reached 0.5. Then, the culture medium was left in ice water for 10 minutes, and the culture medium was centrifuged with 7000G at 4°C for 10 minutes. 15mL of sucrose (270mM) and 0.11mL of NaH2PO4(686mM, pH 7.4) were mixed with each other, thereby preparing an electroporation buffer solution. After a cell pellet was washed with the electroporation buffer solution prepared as described above three times, the washed cell pellet was suspended in 2mL of the same buffer solution, thereby constructing recombinant cells. 0.5 to 2.0 µg of plasmid containing secondary alcohol dehydrogenase gene was added to the prepared cells (500 µl)for transformation, and then electroporation (4mm cuvette, 2.5kV, ∞Ω, 25uF) was performed using Gene pulser II (Bio-Rad Corp.), followed by anaerobic culture in the culture medium to which antibiotic was added, thereby completing the construction of recombinant strain. All of the plasmids used for transformation were constructed so as not to be affected by restriction system of the *Clostridium* strain by being methylated in *E. coli* ER2275 transformed with a pAN1 vector before electroporation.

The *Clostridium* strain PJC4BK-IPA2 cultured as described above was smeared on a CGM/chlorampenicol plate medium and anaerobically cultured overnight at 37°C. 2 cultured colonies were inoculated in a 50mL disposable tube in which 20mL of CGM/chlorampenicol culture medium was contained and anaerobically cultured while being placed at 37°C until OD600 reached 3. The cultured broth was inoculated again in a liquid CGM containing 100mL of 8% glucose and anaerobically cultured while being placed at 37°C until OD600 reached 2 to 3. Then, the resultant was inoculated in a culture bath in which 1L of the liquid CGM and 0mL (0g/L, dried weight), 200mL (60g/L), 250mL (75g/L), 300mL (90g/L), 350mL (105g/L), and 400mL(120g/L) of the adsorbent were contained, respectively, and cultured. After starting the culture, concentrations of the produced butanol, acetone, isopropanol, ethanol, and the like, were analyzed per 3 hours while maintaining a concentration of glucose at 20g/L or more. Analysis of products such as butanol, acetone, isopropanol, ethanol, and the like, was performed using the gas chromatography (Agilent, USA), and analysis conditions were the same as in Table 1.

Concentrations of sugar and organic acid may be confirmed using high pressure liquid chromatography (HPLC), gas chromatography, and a sugar analyzer after centrifuging the culture medium and obtaining a supernatant. Conditions of the HPLC were as follows: water containing 0.01N sulfuric acid was used as a mobile phase, and a flow rate was 0.6mL/min. As the column, Aminex87H and Aminex87P (Bio-rad, USA) were used, and the produced sugar and organic acid were analyzed using a reflective index (RI) detector.

As a result, it may be confirmed that the recombinant and mutant strains cultured as described above produced larger amounts of products such as butanol, acetone, isopropanol, ethanol, and the like in the culture medium containing the adsorbent, and the optimal amount of the adsorbent was 250mL/L, as shown in Table 2.

### Table 2

**[Table 2]**

| Amount of adsorbent (mL/L) | product (g/L) | | | | | Yield (%) | Production rate (g/L/h) | Consumption amount of glucose (g/L) |
|---|---|---|---|---|---|---|---|---|
| | Acetone | Ethan ol | Butanol | IPA | Sum | | | |
| 0 | 0.4 | 4.1 | 16.8 | 3.3 | 25 | 35 | 0.6 | 70 |
| 200 | 0.6 | 7.7 | 23.1 | 9.6 | 40 | 33 | 1.35 | 120 |
| 250 | 3.6 | 8.4 | 27.6 | 6.2 | 46 | 34 | 1.2 | 132 |
| 300 | 2.3 | 6.2 | 27.1 | 7.8 | 43.3 | 30 | 1.09 | 153 |
| 350 | 7.0 | 7.2 | 25.1 | 5.4 | 44.7 | 28 | 1.06 | 161 |
| 400 | 6.4 | 8.53 | 27.2 | 7.6 | 49.7 | 30 | 1.10 | 166 |

### <Experimental Example 4>

The same culture medium, culture method, and analysis method as in the Experimental Examples were used, and 200mL of the adsorbent was used, such that reusability of the adsorbent was tested. After completing the culture, the products such as butanol, acetone, isobutanol, or ethanol, adsorbed in the adsorbent were recovered using a column for desorption and eluted by flowing a tetrahydrofuran solvent having a double volume of a volume of the adsorbent at a flow rate of 10mL/min. Then, the total amount of products was analyzed using gas chromatography, and the total amount of adsorbed solvent was compared thereto every time, thereby analyzing performance of the adsorbent.

As a result, it was confirmed that even though the adsorbent was reused, adsorption of the product was suitably performed (Table 3).

### Table 3

**[Table 3]**

| The number of reuse | Product (g/L) | | | | | Yield (%) | Producti on rate (g/L/h) | Culture time (h) | Consumption amount of glucose (g/L) |
|---|---|---|---|---|---|---|---|---|---|
| | Acetone | Ethanol | Butanol | IPA | Sum | | | | |
| 1 | 2.2 | 6.6 | 26.9 | 6.6 | 42 | 36 | 1.02 | 41 | 117 |
| 2 | 1.8 | 6.4 | 20.0 | 7.7 | 36 | 35 | 1.20 | 30 | 104 |
| 3 | 0.6 | 7.7 | 23.1 | 9.6 | 40 | 33 | 1.35 | 30 | 120 |
| 4 | 1.8 | 7.1 | 23.2 | 7.6 | 40 | 33 | 1.32 | 30 | 121 |
| 5 | 1.7 | 4.7 | 23.4 | 6.5 | 37 | 34 | 1.23 | 30 | 110 |
| 6 | 1.5 | 7.3 | 22.9 | 9.0 | 41 | 37 | 1.03 | 40 | 110 |
| 7 | 1.4 | 6.9 | 22.3 | 8.5 | 39 | 33 | 1.02 | 39 | 118 |
| 8 | 3.3 | 5.6 | 21.4 | 5.5 | 36 | 32 | 1.00 | 36 | 110 |
| 9 | 1.5 | 6.7 | 20.9 | 7.1 | 36 | 32 | 1.01 | 36 | 112 |
| 10 | 2.1 | 6.3 | 22.9 | 6.5 | 38 | 36 | 1.26 | 36 | 104 |

### <Experimental Example 5> Preparation of products such as butanol, acetone, isopropanol, ethanol, and the like, using fed-batch culture method

Based on the results optimized in the Experimental Examples, products such as butanol, acetone, isopropanol, ethanol, and the like, were prepared using a fed-batch culture method. Experimental methods and analysis were performed similarly to those in <Example 3>, but fermentation of glucose contained in the culture medium was performed in a state in which the concentration of the CGM culture medium containing 250mL of the adsorbent was the same or increased 2 times as compared to <Experimental Example 3>, respectively, such that amounts of the prepared products such as butanol, acetone, isopropanol, ethanol, and the like, were compared with each other.

As a result, it may be confirmed that the recombinant and mutant strains cultured as described above produced larger amounts of butanol, acetone, isopropanol, ethanol, and the like, during the culture process, depending on an amount of a nutrient, as shown in Table 4. This result means that in the case of simultaneously injecting the nutrient and carbon source according to the culture method in Example of the present invention, the high concentration products such as butanol, acetone, isopropanol, ethanol, and the like, may be synthesized with a high yield in the continuous culture as well as the fed-batch culture.

### Table 4

**[Table 4]**

| Culture condition | | Product (g/L) | | | | | Solvent increase rate (%) | Yield (%) | Productio n rate (g/L/h) |
|---|---|---|---|---|---|---|---|---|---|
| adsorb ent | Concen tration of CGM | Acetone | Ethanol | Butanol | IPA | Sum | | | |
| O | 2 | 0.77 | 9.70 | 32.4 | 9.06 | 52 | 108 | 33 | 1.24 |
| O | 1 | 3.63 | 8.4 | 27.6 | 6.2 | 46 | 84 | 35 | 1.21 |
| X | 1 | 0.43 | 4.1 | 16.8 | 3.3 | 25 | 0 | 35 | 0.6 |

### <Experimental Example 6> Manufacturing of fed-batch culture apparatus and preparation of products such as butanol, acetone, ethanol, and the like, using culture method

The fed-batch culture apparatus shown in FIG. 1 was manufactured. In this case, two columns were included therein. In order to prevent the adsorbent from being eluted at upper and lower portions of the column having a volume of 500mL to thereby be lost, a filter (about 150 µm) was mounted, and then a stirrer was mounted. Thereafter, 300mL of adsorbent SP850 was filled in two columns (referred to as first and second columns, respectively). These columns were connected to a culture bath using a silicon tube, and a pump was mounted so as to circulate a culture medium in the column. 4-way valves were mounted at an inlet and an outlet of the column so as to flow an eluant when the products such as butanol, acetone, ethanol, and the like were sufficiently adsorbed in the adsorbent in the column during the culture process to thereby perform desorption in real time. When the product was sufficiently adsorbed in the first column, the flow of the culture medium and the product were changed to the second column by adjusting the 4-way value. In the first column in which the flow of the culture medium and the product was blocked, desorption proceeded in a state in which the adsorbent in the first column was not picked out. While desorption proceeded in the first column, adsorption continuously proceeded in the second column to which the culture medium and the product were supplied. Similarly, when the product was sufficiently adsorbed in the second column, the flow of the culture medium and the product was changed to the first column in which desorption was completed by adjusting the 4-way value. The adsorbent in the first and second columns may be reused by repeating the above-mentioned processes. A circulation direction of the culture medium is a direction from the upper portion of the column to the lower portion thereof, but the circulation direction did not matter.

Meanwhile, *C. acetobutylicum* PJC4BK strains capable of producing butanol, acetone, ethanol, and the like, were prepared using a fed-batch culture apparatus and cultured as in the Experimental Examples. First, 300mL of seed anaerobically cultured in the liquid CGM overnight was inoculated and cultured into a reactor in which 2.7L of the liquid CGM was contained. In Experimental Examples of the present invention, the seed was cultured by general batch fermentation, but in order to prepare the high concentration products such as butanol, acetone, ethanol, and the like at a higher cell concentration, cell immobilization type culture may be performed. The culture medium passed through the first column via the pump at a flow rate of 30mL/min simultaneously with starting the culture. It was confirmed that the adsorbent SP850 was suspended in the culture medium while the culture medium passed through the first column to form a slurry phase, such that the flow of the culture medium was not blocked by the aggregates of cells, and the culture medium passed through the first column. In addition, immediately before and after the culture medium passed through the first column, culture medium samples were taken, and concentrations of butanol, acetone, ethanol, and the like, were analyzed using gas chromatography.

During the culture process, a concentration of sugar was maintained at 20g/L using HPLC and a sugar analyzer. Amounts of the products such as butanol, acetone, isopropanol, ethanol, or the like were confirmed through gas chromatography.

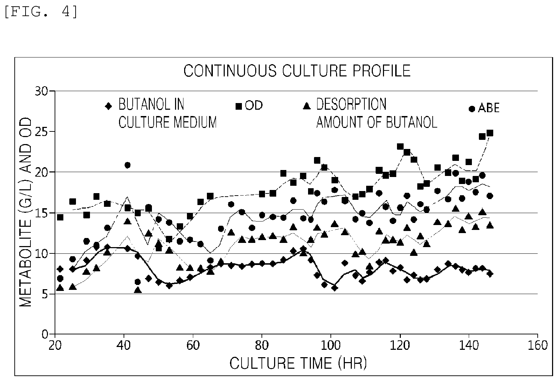

As a result, it was confirmed that the fed-batch culture was stably performed for about 150 hours, and the concentration of the product in the discharge solution (after adsorption) discharged from the column was significantly decreased as compared to the concentration of the product in the culture medium (before adsorption) supplied to the column. That is, it may be confirmed that the products such as butanol, acetone, ethanol, and the like, was maintained at a significantly low concentration in the discharge solution discharged from the column. In addition, it was confirmed that the concentration of the product was constantly maintained in the culture apparatus, such that the microbe was not affected by the toxicity of butanol but may stably prepare the product. Further, the products such as butanol, acetone, ethanol, and the like, adsorbed in the first column were desorbed using water (95°C), and as a result, it was confirmed that the products such as butanol, acetone, ethanol, and the like, were excellently adsorbed and desorbed. Furthermore, it was confirmed that even in the case of repeating replacement of the column 20 times or more, the adsorption performance of the column was not decreased (Tables 5 and 6 and FIG. 4).

### Table 5

**[Table 5]**

| | Product(g) | | | | Yield (%) | Production rate (g/L/h) | Culture time (hour) | Consumption amount of glucose (g) |
|---|---|---|---|---|---|---|---|---|
| | Acetone | Ethanol | Butanol | Sum | | | | |
| Culture medium | 14.1 | 49.9 | 61.8 | 125.8 | 31 | 1.24 | 146 | 2610 |
| Desorption | 58.4 | 106.5 | 525.7 | 690.6 | | | | |
| Sum | 72.4 | 156.4 | 587.6 | 816.4 | | | | |

Results of preparing acetone, ethanol, and butanol

### Table 6

**[Table 6]**

| A | After desorption | | | Acetone | | Ethanol | | Butanol | | B* |
|---|---|---|---|---|---|---|---|---|---|---|
| | Acetone | Ethanol | Butanol | C | D | C | D | C | D | |
| 22 | 1.1 | 0.0 | 5.8 | 0.6 | 3.6 | 1.1 | 2.1 | 0.3 | 8.1 | 1a |
| 25 | 2.5 | 0.9 | 5.9 | 1.1 | 3.6 | 1.6 | 3.0 | 1.6 | 8.0 | 1b |
| 29 | 2.4 | 1.3 | 7.7 | 1.0 | 3.7 | 1.8 | 3.9 | 2.1 | 9.1 | 2a |
| 32 | 1.7 | 1.1 | 8.2 | 1.3 | 4.0 | 2.5 | 5.0 | 2.9 | 10.7 | 2b |
| 35 | 1.7 | 1.3 | 10.1 | 1.6 | 3.8 | 3.6 | 5.4 | 3.8 | 10.8 | 3a |
| 41 | 3.4 | 3.5 | 13.9 | 1.2 | 3.6 | 2.6 | 5.9 | 4.0 | 10.6 | 3b |
| 44 | 0.0 | 0.9 | 5.5 | 0.5 | 3.0 | 2.5 | 5.6 | 0.7 | 9.6 | 4a |
| 47 | 1.5 | 1.7 | 12.4 | 0.2 | 2.8 | 1.3 | 5.8 | 0.6 | 7.0 | 4b |
| 50 | 1.4 | 1.6 | 11.2 | 0.5 | 2.6 | 2.5 | 5.8 | 0.6 | 6.4 | 5a |
| 53 | 1.5 | 1.9 | 10.4 | 0.6 | 2.9 | 2.3 | 6.2 | 0.8 | 6.0 | 5b |
| 56 | 1.4 | 1.8 | 8.2 | 0.4 | 2.8 | 2.4 | 6.6 | 0.5 | 6.7 | 6a |
| 59 | 1.4 | 2.0 | 8.2 | 0.5 | 2.7 | 3.0 | 7.0 | 0.6 | 7.0 | 6b |
| 62 | 1.1 | 1.8 | 8.2 | 0.8 | 2.7 | 4.5 | 7.7 | 1.1 | 8.1 | 7a |
| 65 | 0.2 | 1.1 | 7.7 | 0.5 | 2.6 | 3.4 | 8.2 | 0.5 | 8.2 | 7b |
| 68 | 1.4 | 2.6 | 9.0 | 0.5 | 2.6 | 3.9 | 8.7 | 0.8 | 9.0 | 8a |
| 71 | 1.2 | 2.2 | 12.6 | 0.5 | 2.5 | 3.5 | 8.6 | 0.7 | 8.4 | 8b |
| 74 | 1.3 | 2.1 | 11.6 | 0.2 | 2.5 | 2.3 | 8.7 | 0.3 | 8.4 | 9a |
| 77 | 0.0 | 1.3 | 11.8 | 0.5 | 2.4 | 3.9 | 8.9 | 1.0 | 8.7 | 9b |
| 80 | 0.9 | 1.9 | 11.9 | 0.4 | 2.4 | 3.8 | 8.9 | 0.8 | 8.7 | 10a |
| 83 | 0.7 | 1.7 | 12.2 | 0.6 | 2.4 | 4.5 | 9.1 | 1.0 | 8.7 | 10b |
| 86 | 0.8 | 1.8 | 11.7 | 0.4 | 2.4 | 3.8 | 9.0 | 0.8 | 9.1 | 11a |
| 89 | 1.0 | 2.2 | 13.2 | 0.5 | 2.5 | 4.3 | 10.0 | 0.8 | 10.3 | 11b |
| 92 | 1.1 | 3.1 | 10.1 | 0.9 | 2.3 | 6.4 | 10.2 | 1.7 | 10,6 | 12a |
| 94 | 0.6 | 1.9 | 11.6 | 0.4 | 2.0 | 4.2 | 9.9 | 0.6 | 9.1 | 12b |
| 96 | 1.2 | 3.0 | 13.1 | 0.6 | 1.7 | 5.9 | 9.4 | 1.2 | 7.3 | 13a |
| 98 | 1.1 | 2.9 | 12.3 | 0.3 | 1.6 | 4.0 | 9.1 | 0.6 | 6.2 | 13b |
| 101 | 1.1 | 3.1 | 13.6 | 0.3 | 1.5 | 4.0 | 8.9 | 0.9 | 5.8 | 14a |
| 104 | 1.0 | 2.8 | 12.6 | 0.3 | 1.6 | 3.6 | 8.7 | 0.5 | 8.7 | 14b |
| 107 | 1.3 | 2.9 | 9.9 | 0.2 | 2.1 | 2.3 | 8.9 | 0.3 | 7.2 | 15a |
| 109 | 1.6 | 3.2 | 10.2 | 0.2 | 2.1 | 2.7 | 8.7 | 0.3 | 6.7 | 15b |
| 111 | 1.7 | 3.7 | 8.3 | 0.3 | 2.2 | 2.9 | 8.6 | 0.7 | 7.7 | 16a |
| 114 | 1.6 | 3.1 | 12.7 | 0.3 | 2.2 | 3.2 | 8.7 | 0.8 | 8.9 | 16b |
| 116 | 1.3 | 2.7 | 11.6 | 0.3 | 2.0 | 2.7 | 9.4 | 0.8 | 9.0 | 17a |
| 118 | 0.7 | 1.7 | 11.7 | 0.4 | 1.9 | 3.6 | 8.4 | 0.9 | 7.8 | 17b |
| 120 | 1.2 | 2.9 | 11.3 | 0.2 | 1.9 | 2.1 | 9.1 | 0.8 | 8.3 | 18a |
| 122 | 1.2 | 2.7 | 13.2 | 0.6 | 1.7 | 5.0 | 8.1 | 1.3 | 6.7 | 18b |
| 124 | 1.2 | 2.9 | 10.1 | 0.3 | 1.7 | 3.2 | 8.4 | 0.8 | 7.2 | 19a |
| 126 | 1.1 | 2.7 | 12.2 | 0.3 | 1.7 | 3.4 | 8.2 | 0.7 | 6.8 | 19b |
| 128 | 1.2 | 2.9 | 11.2 | 0.1 | 1.8 | 1.5 | 8.3 | 0.4 | 6.9 | 20a |
| 131 | 1.2 | 2.6 | 13.9 | 0.2 | 2.0 | 2.5 | 8.8 | 0.4 | 8.0 | 20b |
| 134 | 0.9 | 2.1 | 13.7 | 0.3 | 2.2 | 2.7 | 9.0 | 0.6 | 8.7 | 21a |
| 136 | 1.4 | 2.9 | 15.5 | 0.4 | 2.1 | 3.5 | 8.8 | 0.7 | 8.4 | 21b |
| 138 | 1.2 | 2.7 | 12.8 | 0.3 | 2.0 | 3.0 | 8.6 | 0.9 | 7.9 | 22a |
| 140 | 1.3 | 2.9 | 14.5 | 0.6 | 2.0 | 4.7 | 8.5 | 1.1 | 7.6 | 22b |
| 142 | 1.2 | 3.0 | 13.4 | 0.3 | 2.2 | 3.0 | 9.2 | 0.8 | 8.1 | 23a |
| 144 | 1.3 | 2.9 | 15.2 | 0.3 | 2.2 | 3.2 | 9.3 | 0.7 | 8.1 | 23b |
| 146 | 1.1 | 2.3 | 13.5 | 0.3 | 2.2 | 3.0 | 9.0 | 0.8 | 7.6 | 24a |

| | | | | | | | | | | |
|---|---|---|---|---|---|---|---|---|---|---|
| * a and b mean the first and second columns, respectively. Unit: g/L A: Culture time B: The number of replaced column C: After adsorption D: Before adsorption | | | | | | | | | | |

### <Detailed Description of Main Elements>

100: fermentation, separation, and refinement apparatus
110: first column 120: second column
112: adsorbent 114: filter
130: culture bath 140: supply bath
150: storage bath 160: pump
170: first conversion part 172: second conversion part
174: third conversion part 176: fourth conversion part

## Claims

1. A continuous fermentation, separation, and refinement apparatus (100) of products comprising:
a culture bath (130) in which a microbe is cultured together with a raw material to produce the products;
at least two columns (110,120) filled with an adsorbent (112); and
a conversion part (170,172,174,176) configured to control a flow of a culture medium containing the products, so that a culture medium containing the products is supplied from the culture bath (130) to a specific column (110,120),
wherein the conversion part (170,172,174,176) is configured to change into where the culture medium flows from a first column (110) to a second column (120) in the case in which the products are sufficiently adsorbed in the first column (110) supplied with the culture medium, wherein a continuous flow of culture medium from the culture bath (130) is maintained, wherein
the case in which the products are sufficiently adsorbed is a case in which an adsorption rate of the product with respect to the adsorbent is decreased, a case in which a concentration of the product in the discharge solution discharged from the column (110,120) is 80% or more of a concentration of the product in the culture medium supplied to the column (110,120), a case in which growth of the microbe in the culture bath (130) is inhibited by an increase in the concentration of the product, or a case in which productivity of the product of the microbe is decreased, or a case in which the concentration of butanol in the culture medium reaches at about 12 g/L,
and wherein the first column and second column (110,120) each include a stirrer stirring the adsorbent and the product in the column and a filter (114) preventing the adsorbent from being eluted to thereby be lost.

2. The continuous fermentation, separation, and refinement apparatus (100) of claim 1, wherein a type of the column (110,120) to which the culture medium is supplied is a slurry reactor type, a fluidized reactor type, or a packed reactor type.

3. The continuous fermentation, separation, and refinement apparatus (100) of claim 1, further comprising a storage bath (160) storing the product desorbed from the adsorbent.

4. A continuous fermentation, separation, and refinement method of products comprising:
culturing a microbe together with a raw material to produce the products;
supplying a culture medium containing the products to a first column (110) filled with an adsorbent; and
stopping supplying the culture medium to the first column in the case in which the products are sufficiently adsorbed in the first column (110) and supplying the culture medium to a second column (120) wherein a continuous flow of culture medium from the culture bath (130) is maintained,
wherein at least some of the discharge solution discharged from the first column (110) is used in culturing the microbe again, and wherein
the case in which the products are sufficiently adsorbed is a case in which an adsorption rate of the product with respect to the adsorbent is decreased, a case in which a concentration of the product in the discharge solution discharged from the column is 80% or more of a concentration of the product in the culture medium supplied to the column, a case in which growth of the microbe in the culture bath is inhibited by an increase in the concentration of the product, or a case in which productivity of the product of the microbe is decreased, or a case in which the concentration of butanol in the culture medium reaches at about 12 g/L.

5. The continuous fermentation, separation, and refinement method of claim 4, wherein the raw material is fatty acids or sugars.

6. The continuous fermentation, separation, and refinement method of claim 4, wherein the product is alcohol, ketone, ester, or carboxylic acid.

7. The continuous fermentation, separation, and refinement method of claim 4, wherein the adsorbent is filled at a volume fraction of 0.1 to 99% based on a volume of the column.

8. The continuous fermentation, separation, and refinement method of claim 4 further comprising, desorbing the product adsorbed in the adsorbent in the first column (110) to which supply of the culture medium is stopped while adsorption of the product proceeds in the second column (120) to which the culture medium is supplied.

9. The continuous fermentation, separation, and refinement method of claim 4, wherein the desorption is performed using heat or an eluant.

10. The continuous fermentation, separation, and refinement method of claim 4, wherein the desorption is performed by applying heat to the adsorbed product in the adsorbent to vaporize the product.

11. The continuous fermentation, separation, and refinement method of claim 4, wherein the desorption is performed using the eluant, which is an organic solvent, or an acidic or basic aqueous solution.

## Patentansprüche

1. Kontinuierliche Fermentations-, Trenn- und Raffinierungsvorrichtung (100) für Produkte, aufweisend:
ein Kulturbad (130), in dem eine Mikrobe zusammen mit einem Rohmaterial zur Herstellung der Produkte kultiviert wird;
mindestens zwei Säulen (110, 120), die mit einem Adsorptionsmittel (112) gefüllt sind; und
einen Umwandlungsteil (170, 172, 174, 176), der so konfiguriert ist, dass er einen Fluss eines die Produkte enthaltenden Kulturmediums steuert, so dass ein die Produkte enthaltendes Kulturmedium aus dem Kulturbad (130) einer spezifischen Säule (110, 120) zugeführt wird,
wobei der Umwandlungsteil (170, 172, 174, 176) so konfiguriert ist, dass er in den Fall, in dem die Produkte ausreichend in der ersten Säule (110), die mit dem Kulturmedium versorgt wird, adsorbiert werden, in den Bereich wechselt, in dem das Kulturmedium von einer ersten Säule (110) zu einer zweiten Säule (120) fließt, wobei ein kontinuierlicher Fluss des Kulturmediums aus dem Kulturbad (130) aufrechterhalten wird, wobei
der Fall, in dem die Produkte ausreichend adsorbiert sind, ein Fall ist, in dem eine Adsorptionsrate des Produkts in Bezug auf das Adsorptionsmittel vermindert ist, ein Fall, in dem eine Konzentration des Produkts in der aus der Säule (110, 120) ausgetragenen Austragslösung 80% oder mehr einer Konzentration des Produkts in dem der Säule (110, 120) zugeführten Kulturmedium beträgt, ein Fall, in dem das Wachstum der Mikrobe im Kulturbad (130) durch eine Erhöhung der Konzentration des Produkts gehemmt wird, oder ein Fall, in dem die Produktivität des Produkts der Mikrobe vermindert wird, oder ein Fall, in dem die Konzentration von Butanol im Kulturmedium etwa 12 g/L erreicht,
und wobei die erste Säule und die zweite Säule (110, 120) jeweils einen Rührer, der das Adsorptionsmittel und das Produkt in der Säule rührt, und einen Filter (114) aufweisen, der verhindert, dass das Adsorptionsmittel eluiert wird und dadurch verloren geht.

2. Die kontinuierliche Fermentations-, Trenn- und Raffinierungsvorrichtung (100) nach Anspruch 1, wobei ein Typ der Säule (110, 120), der das Kulturmedium zugeführt wird, ein Aufschlämmungsreaktortyp, ein fluidisierter Reaktortyp oder ein gepackter Reaktortyp ist.

3. Die kontinuierliche Fermentations-, Trenn- und Raffinierungsvorrichtung (100) nach Anspruch 1, die ferner ein Speicherbad (160) aufweist, in dem das vom Adsorptionsmittel desorbierte Produkt gelagert ist.

4. Verfahren zum kontinuierlichen Fermentieren, Trennen und Raffinieren von Produkten, aufweisend:
Kultivieren einer Mikrobe zusammen mit einem Rohmaterial zur Herstellung der Produkte;
Zuführen eines die Produkte enthaltendes Kulturmediums zu einer ersten Säule (110), die mit einem Adsorptionsmittel gefüllt ist; und
Stoppen der Zufuhr des Kulturmediums zu der ersten Säule in dem Fall, in dem die Produkte in der ersten Säule (110) ausreichend adsorbiert sind, und Zufuhr des Kulturmediums zu einer zweiten Säule (120), in der ein kontinuierlicher Fluss des Kulturmediums aus dem Kulturbad (130) aufrechterhalten wird,
wobei mindestens ein Teil der aus der ersten Säule (110) ausgetragenen Austragslösung zur erneuten Kultivierung der Mikrobe verwendet wird, und wobei
der Fall, in dem die Produkte ausreichend adsorbiert sind, ist ein Fall, in dem eine Adsorptionsrate des Produkts in Bezug auf das Adsorptionsmittel vermindert ist, ein Fall, in dem eine Konzentration des Produkts in der aus der Säule abgegebenen Austragslösung 80% oder mehr einer Konzentration des Produkts in dem der Säule zugeführten Kulturmedium beträgt, ein Fall, in dem das Wachstum der Mikrobe im Kulturbad durch eine Erhöhung der Konzentration des Produkts gehemmt wird, oder ein Fall, in dem die Produktivität des Produkts der Mikrobe vermindert wird, oder ein Fall, in dem die Konzentration von Butanol im Kulturmedium etwa 12 g/L erreicht.

5. Das Verfahren zum kontinuierlichen Fermentieren, Trennen und Raffinieren nach Anspruch 4, wobei das Rohmaterial Fettsäuren oder Zucker ist.

6. Das Verfahren zum kontinuierlichen Fermentieren, Trennen und Raffinieren nach Anspruch 4, wobei das Produkt Alkohol, Keton, Ester oder Carbonsäure ist.

7. Das Verfahren zum kontinuierlichen Fermentieren, Trennen und Raffinieren nach Anspruch 4, wobei das Adsorptionsmittel mit einem Volumenanteil von 0,1 bis 99%, basierend auf ein Volumen der Säule, gefüllt ist.

8. Das Verfahren zum kontinuierlichen Fermentieren, Trennen und Raffinieren nach Anspruch 4, weiterhin umfassend das Desorbieren des in dem Adsorptionsmittel adsorbierten Produkts in der ersten Säule (110), zu der die Zufuhr des Kulturmediums gestoppt wird, während die Adsorption des Produkts in der zweiten Säule (120), zu der das Kulturmedium zugeführt wird, fortschreitet.

9. Das Verfahren zum kontinuierlichen Fermentieren, Trennen und Raffinieren nach Anspruch 4, wobei die Desorption unter Verwendung von Wärme oder eines Elutionsmittels durchgeführt wird.

10. Das Verfahren zum kontinuierlichen Fermentieren, Trennen und Raffinieren nach Anspruch 4, wobei die Desorption durch Anwendung von Wärme auf das adsorbierte Produkt im Adsorptionsmittel durchgeführt wird, um das Produkt zu verdampfen.

11. Das Verfahren zum kontinuierlichen Fermentieren, Trennen und Raffinieren nach Anspruch 4, wobei die Desorption unter Verwendung des Elutionsmittels, das ein organisches Lösungsmittel oder eine saure oder basische wässrige Lösung ist, durchgeführt wird.

## Revendications

1. Appareil de fermentation, séparation, et raffinage continu (100) de produits comprenant :
un bain de culture (130) dans lequel un microbe est mis en culture avec une matière première pour produire les produits ;
au moins deux colonnes (110, 120) remplies d'un adsorbant (112) ; et
une partie de conversion (170, 172, 174, 176) configurée pour contrôler un écoulement d'un milieu de culture contenant les produits, de sorte qu'un milieu de culture contenant les produits est introduit à partir du bain de culture (130) dans une colonne spécifique (110, 120),
dans lequel la partie de conversion (170, 172, 174, 176) est configurée pour transformer où le milieu de culture s'écoule à partir d'une première colonne (110) dans une seconde colonne (120) dans le cas où les produits sont suffisamment adsorbés dans la première colonne (110) alimentée avec le milieu de culture, dans lequel un écoulement continu de milieu de culture à partir du bain de culture (130) est maintenu, dans lequel
le cas dans lequel les produits sont suffisamment adsorbés est un cas dans lequel une vitesse d'adsorption du produit par rapport à l'adsorbant diminue, un cas dans lequel une concentration du produit dans la solution de décharge déchargée à partir de la colonne (110, 120) est de 80 % ou plus d'une concentration du produit dans le milieu de culture introduit dans la colonne (110, 120), un cas dans lequel la croissance du microbe dans le bain de culture (130) est inhibée par une augmentation de la concentration du produit, ou un cas dans lequel la productivité du produit du microbe diminue, ou un cas dans lequel la concentration en butanol dans le milieu de culture atteint environ 12 g/l,
et dans lequel les première colonne et seconde colonne (110, 120) comprennent chacune un agitateur agitant l'adsorbant et le produit dans la colonne et un filtre (114) évitant que l'adsorbant ne soit élué pour être par-là perdu.

2. Appareil de fermentation, séparation, et raffinage continu (100) selon la revendication 1, dans lequel un type de la colonne (110, 120) dans laquelle le milieu de culture est introduit est un type de réacteur à suspension, un type de réacteur fluidisé, ou un type de réacteur à garnissage.

3. Appareil de fermentation, séparation, et raffinage continu (100) selon la revendication 1, comprenant de plus un bain de stockage (160) stockant le produit désorbé de l'adsorbant.

4. Procédé de fermentation, séparation, et raffinage continu de produits comprenant :
la mise en culture d'un microbe avec une matière première pour produire les produits ;
l'introduction d'un milieu de culture contenant les produits dans une première colonne (110) chargée d'un adsorbant ; et
l'interruption d'introduction du milieu de culture dans la première colonne dans le cas où les produits sont suffisamment adsorbés dans la première colonne (110) et d'introduction du milieu de colonne dans une seconde colonne (120) dans laquelle un écoulement continu de milieu de culture à partir du bain de culture (130) est maintenu,
dans lequel au moins une certaine quantité de la solution de décharge déchargée à partir de la première colonne (110) est utilisée à nouveau dans une mise en culture du microbe, et dans lequel
le cas dans lequel les produits sont suffisamment adsorbés est un cas dans lequel une vitesse d'adsorption du produit par rapport à l'adsorbant diminue, un cas dans lequel une concentration du produit dans la solution de décharge déchargée de la colonne est de 80 % ou plus d'une concentration du produit dans le milieu de culture introduit dans la colonne, un cas dans lequel la croissance du microbe dans le bain de culture est inhibée par une augmentation de la concentration du produit, ou un cas dans lequel la productivité du produit du microbe diminue, ou un cas dans lequel la concentration en butanol dans le milieu de culture atteint environ 12 g/l.

5. Procédé de fermentation, séparation, et raffinage continu selon la revendication 4, dans lequel la matière première est des acides gras ou des sucres.

6. Procédé de fermentation, séparation, et raffinage continu selon la revendication 4, dans lequel le produit est un alcool, une cétone, un ester, ou un acide carboxylique.

7. Procédé de fermentation, séparation, et raffinage continu selon la revendication 4, dans lequel l'adsorbant est chargé à une fraction de volume de 0,1 à 99 % sur la base d'un volume de la colonne.

8. Procédé de fermentation, séparation, et raffinage continu selon la revendication 4, comprenant de plus, la désorption du produit adsorbé dans l'adsorbant dans la première colonne (110) dans laquelle l'introduction du milieu de culture est interrompue pendant que l'adsorption du produit a lieu dans la seconde colonne (120) dans laquelle le milieu de culture est introduit.

9. Procédé de fermentation, séparation, et raffinage continu selon la revendication 4, dans lequel la désorption est réalisée en utilisant de la chaleur ou un éluant.

10. Procédé de fermentation, séparation, et raffinage continu selon la revendication 4, dans lequel la désorption est réalisée par application de chaleur au produit adsorbé dans l'adsorbant pour vaporiser le produit.

11. Procédé de fermentation, séparation, et raffinage continu selon la revendication 4, dans lequel la désorption est réalisée en utilisant l'éluant, qui est un solvant organique ou une solution aqueuse acide ou basique.
